(19) Europäisches Patentamt
European Patent Office
Office européen des brevets

(11) EP 2 123 643 A1

(12) **EUROPEAN PATENT APPLICATION**
published in accordance with Art. 153(4) EPC

(43) Date of publication:
**25.11.2009 Bulletin 2009/48**

(21) Application number: **08722106.5**

(22) Date of filing: **14.03.2008**

(51) Int Cl.:
***C07D 265/30*** *(2006.01)* ***A61K 31/5375*** *(2006.01)*
***A61P 3/10*** *(2006.01)* ***A61P 25/04*** *(2006.01)*

(86) International application number:
**PCT/JP2008/054710**

(87) International publication number:
**WO 2008/111669 (18.09.2008 Gazette 2008/38)**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **15.03.2007 JP 2007066727**
**19.09.2007 JP 2007242210**

(71) Applicant: **Astellas Pharma Inc.**
**Tokyo 103-8411 (JP)**

(72) Inventors:
- **AOKI, Toshiaki**
  **Tokyo 103-8411 (JP)**
- **MURAI, Nobuhito**
  **Tokyo 103-8411 (JP)**
- **TAMURA, Seiji**
  **Tokyo 103-8411 (JP)**
- **YAMAMOTO, Hiroko**
  **Tokyo 103-8411 (JP)**
- **HAMAKAWA, Nozomu**
  **Tokyo 103-8411 (JP)**

(74) Representative: **Albrecht, Thomas et al**
**Kraus & Weisert**
**Patent- und Rechtsanwälte**
**Thomas-Wimmer-Ring 15**
**D-80539 München (DE)**

(54) **NOVEL PROPHYLACTIC AND/OR THERAPEUTIC AGENT FOR DIABETIC NEUROPATHY**

(57) The present invention relates to an agent for preventing and/or treating diabetic neuropathy comprising a 2-[(substituted-inden-7-yloxy)methyl]morpholine of the formula (I) or a pharmaceutically acceptable salt thereof as an active ingredient. The invention is useful for providing an excellent agent for preventing and/or treating diabetic neuropathy, and particularly useful for providing an agent for preventing and/or treating diabetic motor neuropathy (such as muscle weakness disorder (such as muscle weakness disorder with inability to walk independently)), diabetic sensory neuropathy (such as paresthesia (such as vibration perception abnormality), allodynia, hypoesthesia (such as numbness of extremities or cold sensation), or pain), or diabetic autonomic neuropathy (such as stool abnormality (such as constipation or diarrhea), urination disorder, impotence, orthostatic hypotension, sudomotor dysfunction, abnormal heart rate variability, or delayed gastric emptying). Further, the invention is particularly useful for providing an agent for improving pathophysiology of diabetic neuropathy.

EP 2 123 643 A1

## Description

Technical Field

The present invention relates to a novel pharmaceutical use of a morpholine derivative or a pharmaceutically acceptable salt thereof as a preventive and/or therapeutic agent for diabetic neuropathy.

Background Art

Among diabetic complications, retinopathy (eye), neuropathy (nerve), and nephropathy (kidney) are known as three major complications and are big problems in clinical practice.

According to Thomas et al. (Diabetes, vol. 46, Suppl. 2, pp. S54-S57, 1997), diabetic neuropathy includes polyneuropathy and mononeuropathy. Polyneuropathy includes sensory neuropathy, motor neuropathy, and autonomic neuropathy each of which is peripheral neuropathy. In diabetic sensory neuropathy, paresthesia such as numbness or pain is observed as acute pathophysiology, and hypoesthesia (such as numbness of extremities, vibration perception, or cold sensation), pain or the like is observed as chronic pathophysiology. Further, as diabetic motor neuropathy, muscle weakness with inability to walk independently can be exemplified. Further, in diabetic autonomic neuropathy, an abnormality may be caused in any organ regulated by the autonomic nerves, and stool abnormality such as constipation or diarrhea, urination disorder, impotence, orthostatic hypotension, sudomotor dysfunction, delayed gastric emptying or the like is observed. On the other hand, mononeuropathy includes disorders of central nervous system such as brain and peripheral neuropathy, and peripheral mononeuropathy includes external ophthalmoplegia (Diabetes vol. 46, Suppl. 2, pp. S54-S57, 1997, and Muscle Nerve, vol. 11, pp. 21-32, 1988).

The progress of pathological conditions of polyneuropathy is slow, and metabolic abnormalities accompanying diabetes are thought to be a major cause. As a causal factor, not only a single factor is involved, but a plurality of factors are closely related to one another to exacerbate the pathophysiology. That is, due to a hyperglycemic state, a polyol metabolic pathway is activated and conversion of glucose into sorbitol is increased, resulting in a decrease in NADPH. It is known that this decreases blood flow in the nerve or increases oxidative stress. Further, due to sorbitol accumulated in the nerve, functional and structural neuropathy is induced. Further, it is known that a neurofilament, a myelin sheath protein, an extracellular matrix protein, or the like are impaired due to excessive glycation of the protein to cause an abnormality of a nerve function and also a repair function of nerve regeneration or the like is impaired to exacerbate pathological conditions (Current Opinion in Neurology, vol. 18, October, pp. 586-590, 2005, and The Informed Prescriber, vol. 11, December, pp. 122-125, 1996).

For diabetic neuropathy, palliative drug therapy against each symptom has been made, for example, a neuropathic pain drug against pain symptoms has been used so far. For example, excess sorbitol generated by conversion of glucose into sorbitol with an aldose reductase is part of the cause of the onset of diabetic complications such as diabetic neuropathy. Therefore, an aldose reductase inhibitor has been developed for the purpose of alleviating diabetic neuropathy, and Epalrestat (manufactured by Ono Pharmaceutical Co., Ltd.) has been approved and used in Japan. However, various problems have been pointed out as to efficacy and side effects of this medicinal agent (The Informed Prescriber, vol. 11, December, pp. 122-125, 1996). Further, it is known that a selective serotonin and norepinephrine reuptake inhibitor (SNRI), (S)-N-methyl-$\gamma$-(1-naphthalenyloxy)-2-thiophenepropanamine(duloxetine) has a beneficial analgesic effect on tonic pain in diabetic neuropathy (Patent document 1). However, no medicinal agents, which has a confirmatory therapeutic effect on the underlying pathophysiology, has been developed yet.

On the other hand, ($\pm$)-2-[(inden-7-yloxy)methyl]morpholine hydrochloride (indeloxazine hydrochloride) had been used for the treatment of psychiatric symptoms in patients with cerebrovascular disorder in Japan and South Korea (Patent document 1 and Non-patent document 3). In rat studies, it has a high affinity for a serotonin and norepinephrine uptake site and is known to have a serotonin and norepinephrine reuptake inhibitory activity in brain and an antidepressive activity. Further, its optically active substances, (+)-2-[(inden-7-yloxy)methyl]morpholine hydrochloride and (-)-2-[(inden-7-yloxy)methyl]morpholine hydrochloride are known to also exhibit a serotonin and norepinephrine reuptake inhibitory activity in the same manner as ($\pm$)-2-[(inden-7-yloxy)methyl]morpholine hydrochloride (Non-patent document 2). Further, it is known that (S)-2-{[(7-fluoroindan-4-yl)oxy]methyl}morpholine hydrochloride has an activity of enhancing neurotransmission by norepinephrine because of having both of an activity of enhancing neurotransmission by serotonin based on a serotonin reuptake inhibitory activity and 5-HT2A receptor antagonism (Non-patent document 3 and Non-patent document 4), and is useful as a therapeutic agent for anxiety or depression and an agent for recovery from dysfunction after onset of cerebral infarction (Patent documents 3 and 4). However, it has not been reported that a morpholine derivative including indeloxazine is effective in diabetic neuropathy.

Patent document 1: WO 00/15223

Patent document 2: US Patent No. 4109088
Patent document 3: US Patent No. 5521180
Patent document 4: US Patent Application Publication No. 2007/0259865
Non-patent document 1: Neuropharmacology, vol. 37, pp. 1169-1176, 1998
Non-patent document 2: Chemical and Pharmaceutical Bulletin, vol. 33, No. 9, pp. 3766-3774, 1985
Non-patent document 3: European Journal of Pharmacology, vol. 395, No. 1, pp. 31-36, 2000
Non-patent document 4: The Journal of Pharmacology and Experimental Therapeutics, vol. 302, No. 3, pp. 983-991, 2002

Disclosure of the invention

Problems to be solved by the invention

An object of the present invention is to provide a novel and excellent agent for preventing and/or treating diabetic neuropathy.

Means for Solving the Problems

The present inventors studies based on their own conception for achieving the above object and found that a morpholine derivative of the invention exhibits an excellent therapeutic effect on diabetic neuropathy and also exhibits an effect of fundamentally improving pathophysiology of diabetic neuropathy, and thus, the invention has been completed.
An object of the invention is to provide an agent for preventing and/or treating diabetic neuropathy comprising the morpholine derivative or a pharmaceutically acceptable salt thereof as an active ingredient.
Another object of the invention is to provide a pharmaceutical composition comprising an effective amount of the morpholine derivative or a pharmaceutically acceptable salt thereof and a pharmaceutically acceptable carrier for preventing and/or treating diabetic neuropathy.
Still another object of the invention is to provide use of the morpholine derivative or a pharmaceutically acceptable salt thereof for manufacture of a medicament for preventing and/or treating diabetic neuropathy.
Still another object of the invention is to provide a method for preventing and/or treating diabetic neuropathy comprising administering an effective amount of the morpholine derivative or a pharmaceutically acceptable salt thereof.
Still another object of the invention is to provide a method for producing a pharmaceutical composition for preventing and/or treating diabetic neuropathy comprising mixing the morpholine derivative or a pharmaceutically acceptable salt thereof with a pharmaceutically acceptable excipient.
Still another object of the invention is to provide a commercial package comprising a pharmaceutical composition containing the morpholine derivative or a pharmaceutically acceptable salt thereof as an active ingredient, and a description that the morpholine derivative or a pharmaceutically acceptable salt thereof can be used or should be used for preventing and/or treating diabetic neuropathy.
The invention relates to an agent for preventing and/or treating diabetic neuropathy comprising a morpholine derivative represented by the formula (I) or a pharmaceutically acceptable salt thereof as an active ingredient.

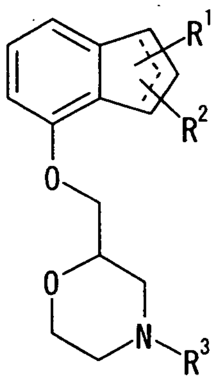

(I)

$R^1$ and $R^2$ are the same or different and represent a hydrogen atom, a lower alkyl , or a phenyl;
$R^3$ represents hydrogen, a lower alkyl , a phenyl , or a benzyl ; and
a dotted line indicates that a double bond can be formed.

Further, the invention relates to an agent for preventing and/or treating diabetic neuropathy comprising (±)-2-[(inden-7-yloxy)methyl]morpholine or a pharmaceutically acceptable salt thereof as an active ingredient.
Further, the invention relates to an agent for preventing and/or treating diabetic neuropathy comprising (+)-2-[(inden-7-yloxy)methyl]morpholine or a pharmaceutically acceptable salt thereof as an active ingredient.
Further, the invention relates to an agent for preventing and/or treating diabetic neuropathy comprising (-)-2-[(inden-7-yloxy)methyl]morpholine or a pharmaceutically acceptable salt thereof as an active ingredient.
Further, the invention relates to an agent for improving pathophysiology of diabetic neuropathy comprising a morpholine derivative represented by the above-mentioned formula (I) or a pharmaceutically acceptable salt thereof as an active ingredient.
Further, the invention relates to an agent for improving pathophysiology of diabetic neuropathy comprising (±)-2-[(inden-7-yloxy)methyl]morpholine or a pharmaceutically acceptable salt thereof as an active ingredient.
Further, the invention relates to an agent for improving pathophysiology of diabetic neuropathy comprising (+)-2-[(inden-7-yloxy)methyl]morpholine or a pharmaceutically acceptable salt thereof as an active ingredient.
Further, the invention relates to an agent for improving pathophysiology of diabetic neuropathy comprising (-)-2-[(inden-7-yloxy)methyl]morpholine or a pharmaceutically acceptable salt thereof as an active ingredient.

Effect of the Invention

The present invention is useful for providing an excellent preventive and/or therapeutic agent for diabetic neuropathy. Further, the invention is particularly useful for providing a preventive and/or therapeutic agent for diabetic motor neuropathy (such as muscle weakness disorder (such as muscle weakness disorder with inability to walk independently)), diabetic sensory neuropathy (such as paresthesia (such as vibration perception abnormality), allodynia, hypoesthesia (such as numbness of extremities or cold sensation), or pain), or diabetic autonomic neuropathy (such as stool abnormality (such as constipation or diarrhea), urination disorder, impotence, orthostatic hypotension, sudomotor dysfunction, abnormal heart rate variability, or delayed gastric emptying). Further, the invention is particularly useful for providing an agent for improving pathophysiology of diabetic neuropathy.

Brief Description of the Drawings

[Fig. 1] It shows an effect of improving paresthesia of (±)-2-[(inden-7-yloxy)methyl]morpholine hydrochloride on rat models of STZ-induced diabetes. The withdrawal threshold (g) along the vertical axis indicates a pain threshold (g) in each group. The symbol "*" in the graph indicates that as a result of the Student's t-test, there is a significant difference compared with the vehicle group at a significance level less than 5%. The symbol "**" in the graph indicates that as a result of the Student's t-test, there is a significant difference compared with the vehicle group at a significance level less than 1%. The symbol "###" in the graph indicates that as a result of the Student's t-test, there is a significant difference compared with the Normal group at a significance level less than 0.5%. a) shows data obtained on day 28 of repeated administration; and b) shows data obtained on day 7 of cessation of the drug after 28 days of repeated administration. The equation "n = 12-14" indicates that each group consisted of 12 to 14 rats.
[Fig. 2] It shows an effect of improving paresthesia of (+)-2-[(1H-inden-7-yloxy)methyl]morpholine hydrochloride on rat models of STZ-induced diabetes. The withdrawal threshold (g) along the vertical axis indicates a pain threshold (g) in each group. The symbol "***" in the graph indicates that as a result of the Student's t-test, there is a significant difference compared with the vehicle group at a significance level less than 0.5%. The symbol "###" in the graph indicates that as a result of the Student's t-test, there is a significant difference compared with the Normal group at a significance level less than 0.5%. a) shows data obtained on day 28 of repeated administration; and b) shows data obtained on day 7 of cessation of the drug after 29 days of repeated administration. The equation "n = 13-14" indicates that each group consisted of 13 to 14 rats.
[Fig. 3] It shows an effect of improving paresthesia of duloxetine on rat models of STZ-induced diabetes. The withdrawal threshold (g) along the vertical axis indicates a pain threshold (g) in each group. The symbol "**" in the graph indicates that as a result of the Student's t-test, there is a significant difference compared with the vehicle group at a significance level less than 1.0%. The symbol "###" in the graph indicates that as a result of the Student's t-test, there is a significant difference compared with the Normal group at a significance level less than 0.1%. a) shows data obtained on day 28 of repeated administration; and b) shows data obtained on day 7 of cessation of the drug after 28 days of repeated administration. The equation "n = 12-14" indicates that each group consisted of 12 to 14 rats.

Best Mode for Carrying Out the Invention

Hereinafter, preferred embodiments of the invention are described.

(1) An agent for preventing and/or treating diabetic polyneuropathy or peripheral mononeuropathy comprising the compound of the formula (I) or a pharmaceutically acceptable salt thereof as an active ingredient.
(2) An agent for preventing and/or treating diabetic motor neuropathy, diabetic sensory neuropathy, or diabetic autonomic neuropathy comprising the compound of the formula (I) or a pharmaceutically acceptable salt thereof as an active ingredient.
(3) An agent for preventing and/or treating muscle weakness disorder accompanying diabetes, paresthesia accompanying diabetes, allodynia accompanying diabetes, hypoesthesia accompanying diabetes, pain accompanying diabetes, stool abnormality such as constipation or diarrhea accompanying diabetes, urination disorder accompanying diabetes, impotence accompanying diabetes, orthostatic hypotension accompanying diabetes, sudomotor dysfunction accompanying diabetes, abnormal heart rate variability accompanying diabetes, delayed gastric emptying accompanying diabetes, or external ophthalmoplegia accompanying diabetes comprising the compound of the formula (I) or a pharmaceutically acceptable salt thereof as an active ingredient.
(4) An agent for improving pathophysiology of motor neuropathy accompanying diabetes, sensory neuropathy accompanying diabetes, or autonomic neuropathy accompanying diabetes comprising the compound of the formula (I) or a pharmaceutically acceptable salt thereof as an active ingredient.

(5) The agent described in (1) wherein the compound of the formula (I) or a pharmaceutically acceptable salt thereof is (±)-2-[(inden-7-yloxy)methyl]morpholine or a pharmaceutically acceptable salt thereof.
(6) The agent described in (2) wherein the compound of the formula (I) or a pharmaceutically acceptable salt thereof is (±)-2-[(inden-7-yloxy)methyl]morpholine or a pharmaceutically acceptable salt thereof.
(7) The agent described in (3) wherein the compound of the formula (I) or a pharmaceutically acceptable salt thereof is (±)-2-[(inden-7-yloxy)methyl]morpholine or a pharmaceutically acceptable salt thereof.
(8) The agent described in (4) wherein the compound of the formula (I) or a pharmaceutically acceptable salt thereof is (±)-2-[(inden-7-yloxy)methyl]morpholine or a pharmaceutically acceptable salt thereof.

(9) The agent described in (1) wherein the compound of the formula (I) or a pharmaceutically acceptable salt thereof is (+)-2-[(inden-7-yloxy)methyl]morpholine or a pharmaceutically acceptable salt thereof.
(10) The agent described in (2) wherein the compound of the formula (I) or a pharmaceutically acceptable salt thereof is (+)-2-[(inden-7-yloxy)methyl]morpholine or a pharmaceutically acceptable salt thereof.
(11) The agent described in (3) wherein the compound of the formula (I) or a pharmaceutically acceptable salt thereof is (+)-2-[(inden-7-yloxy)methyl]morpholine or a pharmaceutically acceptable salt thereof.
(12) The agent described in (4) wherein the compound of the formula (I) or a pharmaceutically acceptable salt thereof is (+)-2-[(inden-7-yloxy)methyl]morpholine or a pharmaceutically acceptable salt thereof.

(13) The agent described in (1) wherein the compound of the formula (I) or a pharmaceutically acceptable salt thereof is (-)-2-[(inden-7-yloxy)methyl]morpholine or a pharmaceutically acceptable salt thereof.
(14) The agent described in (2) wherein the compound of the formula (I) or a pharmaceutically acceptable salt thereof is (-)-2-[(inden-7-yloxy)methyl]morpholine or a pharmaceutically acceptable salt thereof.
(15) The agent described in (3) wherein the compound of the formula (I) or a pharmaceutically acceptable salt thereof is (-)-2-[(inden-7-yloxy)methyl]morpholine or a pharmaceutically acceptable salt thereof.
(16) The agent described in (4) wherein the compound of the formula (I) or a pharmaceutically acceptable salt thereof is (-)-2-[(inden-7-yloxy)methyl]morpholine or a pharmaceutically acceptable salt thereof.

(17) A preferred compound of the morpholine derivative represented by the formula (I):

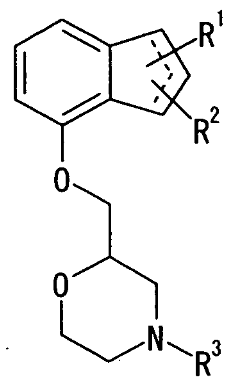

(I)

(wherein $R^1$, $R^2$, and $R^3$ are as defined above, respectively) is a compound of the formula (II):

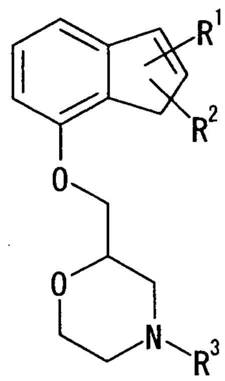

(II)

or the formula (III):

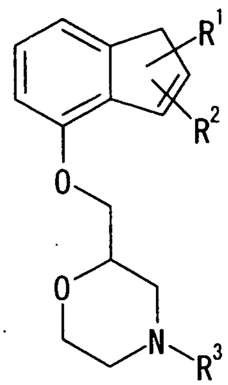

(III)

(wherein $R^1$, $R^2$, and $R^3$ are as defined above, respectively). More preferred is a compound of the formula (II) (wherein $R^1$, $R^2$, and $R^3$ are as defined above, respectively), and further more preferred is a compound of the formula (II) (wherein $R^1$, $R^2$, and $R^3$ represent hydrogen), i.e., (±)-2-[(inden-7-yloxy)methyl]morpholine.

As the pharmaceutically acceptable salt of the morpholine derivative represented by the formula (I) or a pharmaceutically acceptable salt thereof, a hydrochloride is preferred.

(+)-2-[(inden-7-yloxy)methyl]morpholine or a pharmaceutically acceptable salt thereof has an extremely weak CYP inhibitory activity, therefore, this compound is particularly excellent, for example, when it is used for a patient who takes another medicinal agent, because it can be safely administered with little concern of drug interaction.

In the description mentioned above or below of this specification, preferred examples of various definitions included in the scope of the invention are described in detail hereunder.

The "lower alkyl" means a linear or branched aliphatic hydrocarbon having 1 to 6 carbon atoms. Examples thereof include methyl, ethyl, propyl, isopropyl, butyl, isobutyl, tert-butyl, pentyl, and hexyl.

The "diabetic neuropathy" includes diabetic polyneuropathy [diabetic motor neuropathy (such as muscle weakness disorder (such as muscle weakness disorder with inability to walk independently)), diabetic sensory neuropathy (such as paresthesia (such as vibration perception abnormality), allodynia, hypoesthesia (such as numbness of extremities and cold sensation), and pain), and diabetic autonomic neuropathy (such as stool abnormality (such as constipation and diarrhea), urination disorder, impotence, orthostatic hypotension, sudomotor dysfunction, abnormal heart rate variability, and delayed gastric emptying)] which is diabetic peripheral neuropathy, and peripheral mononeuropathy (such as external ophthalmoplegia).

The phrase "improving pathophysiology of diabetic neuropathy" means treating or improving causal pathophysiology of motor, sensory, or autonomic neuropathy by delaying or suppressing progression or exacerbation of neuropathy which is a cause of motor, sensory, or autonomic neuropathy accompanying diabetes, or accelerating repair of the damaged nerve. These are also referred to individually as "improving pathophysiology of diabetic motor neuropathy", "improving pathophysiology of diabetic sensory diabetic neuropathy", and "improving pathophysiology of diabetic autonomic neuropathy", respectively. Specifically, it includes the following phenomenon and the like. An effect of improving motor neuropathy such as difficulty in walking independently due to muscle weakness, sensory neuropathy such as paresthesia (such as vibration perception abnormality), allodynia, hypoesthesia (such as numbness of extremities or cold sensation), or pain, or autonomic neuropathy such as stool abnormality (such as constipation or diarrhea), urination disorder, impotence, orthostatic hypotension, sudomotor dysfunction, or delayed gastric emptying which is observed due to drug therapy for a given period of time is observed continuously even after the drug is eliminated from the body.

The compound of the formula (I) and/or a pharmaceutically acceptable salt thereof can be obtained by the production method described in Patent document 2 and Non-patent document 2 or a production method based on the method.

The compound of the formula (I) may have one or more asymmetric centers, and in that case, it may be present as an enantiomer or a diastereomer. In the invention, a mixture of these isomers and the respective isomers separated from each other are all included.

Accordingly, for example, in addition to (±)-2-[(inden-7-yloxy)methyl]morpholine, its enantiomers, (+)-2-[(inden-7-yloxy)methyl]morpholine and (-)-2-[(inden-7-yloxy)methyl]morpholine are included in the compound of the formula (I).

The compound of the formula (I) can be formed into salts with various acids by a common procedure. The salt of the compound (I) is a pharmaceutically acceptable salt, and examples thereof include organic acid salts (such as acetates, malonates, tartrates, methanesulfonates, benzenesulfonates, formates, toluenesulfonates, and trifluoroacetates), inorganic acid salts (such as hydrochlorides, hydrobromides, sulfates, and phosphates), and amino acid salts (such as alginates, aspartates, and glutamates). Accordingly, the invention includes all pharmaceutically acceptable salts of the morpholine derivatives represented by the formula (I).

The compound of the formula (I) can form hydrates and various pharmaceutically acceptable solvates. These hydrates and solvates are also included in the invention.

A pharmaceutical preparation of the invention can be prepared by a commonly used procedure using a pharmaceutical carrier, excipient, and the like which are commonly used in this field. The administration may be either oral administration of a tablet, a pill, a capsule, a granule, a powder, a liquid, or the like, or parenteral administration by an injection (intraarticular, intravenous, intramuscular, or the like), a suppository, an eye drop, an eye ointment, a transdermal liquid, an ointment, a transdermal adhesive patch, a transmucosal liquid, a transmucosal adhesive patch, an inhalant, or the like.

As a solid composition for oral administration in the invention, a tablet, a powder, a granule, or the like are used. In such a solid composition, one or more active ingredients are mixed with at least one inert diluent such as lactose, mannitol, glucose, hydroxypropyl cellulose, microcrystalline cellulose, starch, polyvinylpyrrolidone, and/or magnesium metasilicate aluminate. The composition may contain an additive other than the inert diluent, for example, a lubricant such as magnesium stearate, a disintegrating agent such as cellulose calcium glycolate, a stabilizing agent, or a solubilizing agent according to a common procedure. The tablet or pill may be coated with a sugar coating of sucrose, gelatin, hydroxypropyl cellulose, hydroxypropylmethyl cellulose phthalate, or the like, or a film of a gastric-soluble or enteric-soluble substance as needed.

A liquid composition for oral administration includes a pharmaceutically acceptable emulsion, solution, suspension, syrup, elixir, and the like, and contains a commonly used inert diluent such as purified water or ethanol. The liquid composition may further contain an auxiliary agent such as a solubilizing agent, a wetting agent, or a suspending agent, a sweetener, a flavor, a perfume, or a preservative other than the inert diluent.

The injection for parenteral administration contains a sterile aqueous or non-aqueous solution, suspension or

emulsion. Examples of the aqueous solution or suspension include distilled water for injection and physiological saline. Examples of the non-aqueous solution or suspension include propylene glycol, polyethylene glycol, vegetable oils such as olive oil, alcohols such as ethanol, and Polysorbate 80 (Pharmacopoeia name). Such a composition may further contain a tonicity agent, a preservative, a wetting agent, an emulsifier, a dispersant, a stabilizing agent, or a solubilizing agent. These are sterilized by, for example, filtration through a bacteria-trapping filter, the addition of a bactericide thereto, or irradiation. Alternatively, a sterile solid composition is prepared, and the resulting composition can be used by being dissolved or suspended in sterile water or a sterile solvent for injection before use.

As a transmucosal preparation such as a transnasal preparation, a solid, a liquid, or a semi-solid preparation are used, and such a preparation can be prepared according to a conventionally known method. For example, a known pH adjusting agent, preservative, thickening agent or excipient are appropriately added and the resulting mixture is formed into a solid, liquid or semi-solid preparation. The transnasal preparation is administered using a common spray apparatus, nasal spray container, tube, intranasal insert, or the like.

A medicinal agent to be used in the invention is administered to a patient with diabetic neuropathy, and a suitable daily dose is, in the case of usual oral administration, from about 0.001 to 100 mg/kg of body weight, preferably from about 0.01 to 100 mg/kg of body weight, more preferably from about 0.01 to 10 mg/kg of body weight. The daily dose is administered once per day or two to four times per day by dividing it into two to four portions. In the case of intravenous administration, a suitable daily dose is from about 0.0001 to 10 mg/kg of body weight, and it is administered once to several times per day by dividing it into one to several portions. Further, in the case of a transmucosal preparation, a dose of about 0.001 to 100 mg/kg of body weight is administered once to several times per day by dividing it into one to several portions. The dose is appropriately determined depending on the individual cases by taking into consideration the symptoms, age, sex, and the like.

## Examples

The following Example is for the purpose of illustrating the present invention in further detail and is not intended to limit the invention. The invention is fully illustrated by way of Examples, however, it will be apparent to those skilled in the art that various modifications and variations can be made in the invention. Accordingly, such modifications and variations are included in the invention as long as they do not depart from the scope of the invention.

### Example 1

STZ (streptozotocin) -induced diabetic rats were prepared according to the following procedure. STZ (45 mg/kg) was intravenously administered to rats at the age of 7 weeks. At week 2 after administration of STZ, the blood was collected from the tail vein and the blood glucose level was measured to confirm that the blood glucose level was increased to 300 mg/dl or more. In the administration group, the rats were grouped so as to minimize the variation in the mean values of body weight, blood glucose level and pain response threshold measured on the previous day of the administration of the drug. A non-administration group of STZ was separately prepared and used as Normal group. A test drug was orally administered once daily for 28 consecutive days. Paresthesia due to neuropathy was determined by a von Frey Test (pain threshold test) at two time points, at 1 hour after administration on day 28 and on day 7 of cessation of the drug after the last administration (Pain, vol. 53, pp. 81-88, 1993). The test was initiated after the rats were placed in a cage for observation and acclimated for at least 20 minutes. By using a digital von Frey Test model 2390 (manufactured by IITC Inc.), a tip end was lightly applied to a paw. When there was no response, an applied load was increased from 1 to 3 g. The pain threshold was determined as an applied load (g) at which an avoidance response such as paw withdrawal was observed. In each animal, the measurement was performed twice for each of the left and right paws, and the mean of the 4 values was calculated as a withdrawal threshold (g). The improvement of paresthesia was determined based on the recovery of the withdrawal threshold in a drug administration group as compared with a vehicle (distilled water) administration group. Statistical test was performed between the vehicle administration group and the drug administration groups. For confirming the onset of diabetic neuropathy, a Student's t-test was also performed to compare the vehicle administration group and the Normal group.

(Results)

The results are shown in Figs. 1 to 3. (±)-2-[(inden-7-yloxy)methyl]morpholine hydrochloride and (+)-2-[(inden-7-yloxy)methyl]morpholine hydrochloride significantly restored the decrease in the withdrawal threshold at a dose of 30 mg/kg on day 28 of repeated administration. That is, since these two compounds exhibited an effect of improving the withdrawal threshold by 28 days of repeated administration, it was shown that these two compounds have an effect of improving the symptoms of paresthesia in diabetic condition. Further, (±)-2-[(inden-7-yloxy)methyl]morpholine hydrochloride and (+)-2-[(inden-7-yloxy)methyl]morpholine hydrochloride also significantly restored the decrease in the withdrawal threshold at a dose of 30 mg/kg even on day 7 of cessation of drug after the repeated administration. That is, since a continuous effect of improving paresthesia was observed even after 7days from when the drug was washed out from the body, it was shown that (±)-2-[(inden-7-yloxy)methyl]morpholine hydrochloride has an effect of not only alleviating the pain symptoms accompanying diabetic neuropathy, but also improving the pathophysiology of diabetic neuropathy. On the other hand, duloxetine exhibited an effect of improving the withdrawal threshold by 28 days of repeated administration, however, it did not exhibit the effect on the decrease in the withdrawal threshold at a dose of 30 mg/kg on day 7 of cessation of drug after the repeated administration.

Example 2

In order to verify whether (+)-2-[(inden-7-yloxy)methyl]morpholine hydrochloride fundamentally cures neuropathy, an improving effect of (+)-2-[(1H-inden-7-yloxy)methyl]morpholine hydrochloride on a decrease in motor nerve conduction velocity (MNCV) in STZ-induced diabetic rats was examined. The measurement was performed using the method of Cameron et al. (The Journal of Experimental Physiology, vol. 74, pp. 917-926, 1989) with some modification. The measurement of MNCV was performed on day 7 to 8 of the drug withdrawal after the repeated daily administration in each of the vehicle administration group and the drug administration group at a dose of 30 mg/kg in Example 2 were used. Further, the same test was performed also for the Normal group. The rats were anesthetized with sodium pentobarbital, and the temperature of the rectum was maintained at about 37.5°C using a body temperature maintenance device for small animals, and then, the MNCV was measured using an evoked potential measurement device. First, a positive stimulating electrode was attached to a dorsal region. The sciatic nerve was exposed, and the nerve around a sciatic notch was directly stimulated (0.1 msec rectangular pulse, 1 Hz, 10 times) with a negative stimulating electrode. Stimulation was adjusted to be just a M-wave amplitude in which maximum response could be obtained . Then, an average waveform evoked by the stimulation was derived from bipolar recording electrodes inserted into a gastrocnemius muscle region of the hind limb of the same side, and the latency (sec) from the start of stimulation to the first deflection of an M-wave was measured. Thereafter, the tibial nerve around behind the knee was stimulated with a negative stimulating electrode in the same manner as above, and the latency (sec) was measured in the same manner as in the gastrocnemius muscle region. A value obtained by dividing a distance (m) between the two regions stimulated with the negative stimulating electrode by a difference (sec) in the M-wave latency measured in the above two stimulation experiments was determined to be an MNCV value. An improving effect on the nerve conductivity was determined based on an improving effect on MNCV in the drug administration group as compared with the vehicle administration group. A significance test was performed between the vehicle group and the drug administration group, and between the vehicle group and the Normal group using a Student's t-test. Each group consisted of 10 to 12 rats.

(Results)

The results of this study is shown in Table 1. (+)-2-[(1H-inden-7-yloxy)methyl]morpholine hydrochloride exhibited a significant improving effect on a decrease in MNCV at a dose of 30 mg/kg. That is, it was revealed that (+)-2-[(1H-inden-7-yloxy)methyl]morpholine hydrochloride exhibited an repairing effect on diabetic neuropathy.
From the above results, it was confirmed that (+)-2-[(1H-inden-7-yloxy)methyl]morpholine hydrochloride has an effect of not only symptomatic relief of paresthesia accompanying diabetic neuropathy, but also curative effects on the pathophysiology of diabetic neuropathy leading to recover the sensory and motor nerve function.

[Table 1]

| | Normal | Vehicle | (+)IDX 30 mg/kg, p.o. |
|---|---|---|---|
| MNCV (m/s) | 62.4±1.7 | 49.4±1.5### | 53.3±1.1* |

The numerical values in the table indicate a mean ± standard error of the mean (SEM) of MNCV.
(+)IDX in the table indicates (+)-2-[(1H-inden-7-yloxy)methyl]morpholine hydrochloride.
The symbol "*" in the table indicates that as a result of the Student's t-test, there is a significant difference compared with the vehicle group at a significance level less than 5%.
The symbol "###" in the table indicates that as a result of the Student's t-test, there is a significant difference compared with the Normal group at a significance level less than 0.5%.
P.O. indicates oral administration.

Example 3

In order to verify whether (+)-2-[(1H-inden-7-yloxy)methyl]morpholine hydrochloride acts on the expression levels of neurotrophic factors, an improving effect of (+)-2-[(inden-7-yloxy)methyl]morpholine hydrochloride on a decrease in the expression levels of neurotrophic factors in the spinal cord and the dorsal root ganglia of STZ-induced diabetic rats was examined.
The expression levels of neurotrophic factors were measured in the animals on day 7 to 8 of cessation of the drug withdrawal after the repeated daily administration in each of the Normal group, the vehicle administration group (STZ-induced diabetic rats) and the drug administration group at a dose of 30 mg/kg (STZ-induced diabetic rats) in Example 2. The spinal cord lumbar region and the dorsal root ganglia (L4, L5, and L6) were excised and the total RNA was extracted from the excised specimens using an RNA extraction kit RNeasy (Qiagen). By using the extracted RNA as a template, in vitro reverse transcription reaction was performed thereby obtaining cDNA. A quantitative PCR analysis was performed for the obtained cDNA using primers to fibroblast growth factor 2 (FGF-2), insulin-like growth factor 1 (IGF-1), and glyceraldehyde-3-phosphate dehydrogenase (G3PDH) by PRISM 7900 (ABI). A data analysis was performed using Sequence Detection System 2.1 (ABI), and the expression levels of FGF-2 and IGF-1 were normalized by the expression level of G3PDH thereby calculating relative gene expression levels. The improving effect on a decrease in the expression levels of neurotrophic factors were determined based on the recovery on the expression levels of neurotrophic factors in the drug administration group as compared with the vehicle administration group. A statistical test was performed between the Normal group and the vehicle group, and between the vehicle group and the drug administration group at a dose of 30 mg/kg using a Student's t-test. Each group consisted of 6 rats.

(Results)

The results of the study is shown in Table 2. (+)-2-[(1H-inden-7-yloxy)methyl]morpholine hydrochloride significantly recovers the decrease in the expression levels of FGF-2 in the spinal cord lumbar region and of IGF-1 in the dorsal root ganglia at a dose of 30 mg/kg. That is, it was revealed that (+)-2-[(1H-inden-7-yloxy)methyl]morpholine hydrochloride restores the decrease in the expression levels of neurotrophic factors in the spinal cord and the dorsal root ganglia in diabetic neuropathy. From the above results, it can be expected that (+)-2-[(1H-inden-7-yloxy)methyl] morpholine hydrochloride has a nerve repairing effect on diabetic neuropathy.

[Table 2]

| | Normal | Vehicle | (+)IDX 30 mg/kg, p.o. |
|---|---|---|---|
| FGF-2 | 100.0±14.9 | 66.9±9.2 | 103.5±4.9** |
| IGF-1 | 100.0±14.1 | 54.6±2.9# | 67.7±3.2* |

The numerical values in the table indicate a mean ± standard error of the mean (SEM) of relative gene expression levels of FGF-2 and IGF-1 normalized by the expression level of G3PDH.
(+)IDX . in the table indicates (+)-2-[(1H-inden-7-yloxy)methyl]morpholine hydrochloride.
The symbols "**" and "*" in the table indicate that as a result of the Student's t-test, there is a significant difference compared with the vehicle group at a significance level less than 1% and 5%, respectively.
The symbol "#" in the table indicates that as a result of the Student's t-test, there is a significant difference compared with the Normal group at a significance level less than 5%.
P.O. indicates oral administration.

Example 4

Test for cytochrome P-450 (1A2) enzyme inhibition using human liver microsomes

An experiment was performed according to the method of Rae Yuan et al. (Drug Metabolism and Disposition, 30(12), 1311-1319, 2002).
By using a 96-well plate, a test drug (0.3 to 10 μmol/L) was added to 100 mmol/L Na-K phosphate buffer (pH = 7.4) containing 0.1 mmol/L ethylenediaminetetraacetic acid (EDTA), 1 mmol/L nicotinamide adenine dinucleotide phosphate (NADPH), and 0.2 mg protein/mL human liver microsomes, and the total volume was made up to 145 μL, and then, preincubation of the resulting mixture was started at 37°C. At 5 minutes after the start of preincubation, phenacetin (20 mmol/L) was added thereto as a substrate for P450(1A2), and the total volume was made up to 150 μL, and then, the resulting mixture was incubated at 37°C for 20 minutes. Thereafter, 130 μL of acetonitrile was added to the sample to

stop the reaction. For the sample in which the reaction was stopped, the concentration of acetaminophen which is a metabolite of phenacetin was determined using LC/MS/MS.
A production amount of acetaminophen in the absence of the test drug was taken as 100%, and a production amount of acetaminophen in the presence of the test drug was calculated for each concentration of the test drug. Based on the calculated production amount for each concentration of the test drug, a concentration of the test drug at which the production amount of acetaminophen was 50% ($IC_{50}$ value) was calculated from the following equation. The test was performed for two cases, and a mean value was used for the calculation of each value.

(Equation)

$$\text{Production amount } [I]\ (\%) = 100 / (1+[I]/IC_{50})$$

Production amount [I] (%): (Production amount of acetaminophen when the test compound concentration was [I]) / (Production amount of acetaminophen in the absence of test compound) x 100

[I]: Test compound concentration

(Results)

The results of the above test are shown in Table 3. There is a large difference between the $IC_{50}$ value shown below and the maximum plasma concentration (Cmax value) at the time of oral administration of (+)-2-[(1H-inden-7-yloxy)methyl]morpholine hydrochloride, therefore, it was revealed that a possibility of drug-drug interaction by oral administration of the compound is extremely low.

[Table 3]

| Compound | $IC_{50}$ (μmol/L) |
|---|---|
| (+)-Indeloxazine hydrochloride | 8.1 |

Industrial Applicability

The pharmaceutical composition of the invention is useful for providing an excellent agent for preventing and/or treating diabetic neuropathy. Further, the invention is particularly useful for providing an agent for preventing and/or treating diabetic motor neuropathy (such as muscle weakness disorder (such as muscle weakness disorder with inability to walk independently)), diabetic sensory neuropathy (such as paresthesia (such as vibration perception abnormality), allodynia, hypoesthesia (such as numbness of extremities or cold sensation), or pain), or diabetic autonomic neuropathy (such as stool abnormality (such as constipation or diarrhea), urination disorder, impotence, orthostatic hypotension, sudomotor dysfunction, abnormal heart rate variability, or delayed gastric emptying). Further, the invention is particularly useful for providing an agent for improving pathophysiology of diabetic neuropathy.

## Claims

1. An agent for preventing and/or treating diabetic neuropathy comprising a compound of the formula (I):

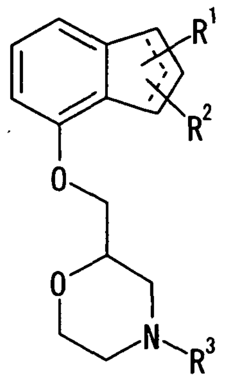

(I)

(wherein $R^1$ and $R^2$ are the same or different and represent a hydrogen atom, a lower alkyl , or a phenyl ; $R^3$ represents hydrogen, a lower alkyl , a phenyl , or a benzyl ; and a dotted line indicates that a double bond can be formed) or a pharmaceutically acceptable salt thereof as an active ingredient.

2. The agent according to claim 1, wherein the compound of the formula (I) or a pharmaceutically acceptable salt thereof is (±)-2-[(inden-7-yloxy)methyl]morpholine or a pharmaceutically acceptable salt thereof.

3. The agent according to claim 1, wherein the compound of the formula (I) or a pharmaceutically acceptable salt thereof is (+)-2-[(inden-7-yloxy)methyl)morpholine or a pharmaceutically acceptable salt thereof.

4. The agent according to claim 1, wherein the compound of the formula (I) or a pharmaceutically acceptable salt thereof is (-)-2-[(inden-7-yloxy)methyl]morpholine or a pharmaceutically acceptable salt thereof.

5. Use of a compound of the formula (I):

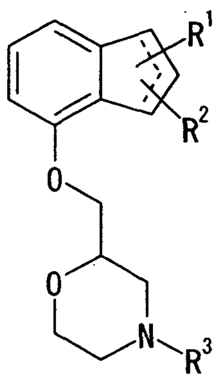

(I)

(wherein $R^1$ and $R^2$ are the same or different and represent a hydrogen atom, a lower alkyl , or a phenyl ; $R^3$ represents hydrogen, a lower alkyl , a phenyl , or a benzyl; and a dotted line indicates that a double bond can be formed) or a pharmaceutically acceptable salt thereof for manufacture of a medicament for preventing and/or treating diabetic neuropathy.

6. The use according to claim 5, wherein the compound of the formula (I) or a pharmaceutically acceptable salt thereof is (±)-2-[(inden-7-yloxy)methyl]morpholine or a pharmaceutically acceptable salt thereof.

7. The use according to claim 5, wherein the compound of the formula (I) or a pharmaceutically acceptable salt thereof is (+)-2-[(inden-7-yloxy)methyl]morpholine or a pharmaceutically acceptable salt thereof.

8. The use according to claim 5, wherein the compound of the formula (I) or a pharmaceutically acceptable salt thereof

is (-)-2-[(inden-7-yloxy)methyl]morpholine or a pharmaceutically acceptable salt thereof.

**9.** A method for preventing and/or treating diabetic neuropathy comprising administering an effective amount of a compound of the formula (I):

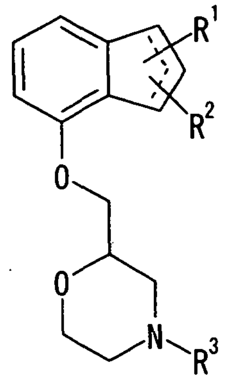

(I)

(wherein $R^1$ and $R^2$ are the same or different and represent a hydrogen atom, a lower alkyl , or a phenyl ;
$R^3$ represents hydrogen, a lower alkyl , a phenyl , or a benzyl ; and
a dotted line indicates that a double bond can be formed) or a pharmaceutically acceptable salt thereof.

**10.** The method according to claim 9, wherein the compound of the formula (I) or a pharmaceutically acceptable salt thereof is (±)-2-[(inden-7-yloxy)methyl]morpholine or a pharmaceutically acceptable salt thereof.

**11.** The method according to claim 9, wherein the compound of the formula (I) or a pharmaceutically acceptable salt thereof is (+)-2-[(inden-7-yloxy)methyl]morpholine or a pharmaceutically acceptable salt thereof.

**12.** The method according to claim 9, wherein the compound of the formula (I) or a pharmaceutically acceptable salt thereof is (-)-2-[(inden-7-yloxy)methyl]morpholine or a pharmaceutically acceptable salt thereof.

**13.** An agent for improving pathophysiology of diabetic neuropathy comprising a compound of the formula (I):

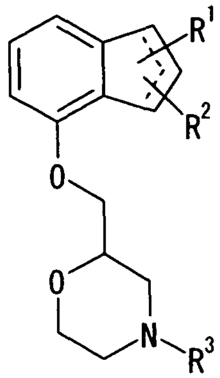

(I)

(wherein $R^1$ and $R^2$ are the same or different and represent a hydrogen atom, a lower alkyl , or a phenyl;
$R^3$ represents hydrogen, a lower alkyl, a phenyl, or a benzyl ; and
a dotted line indicates that a double bond can be formed) or a pharmaceutically acceptable salt thereof as an active ingredient.

**14.** The agent according to claim 13, wherein the compound of the formula (I) or a pharmaceutically acceptable salt thereof is (±)-2-[(inden-7-yloxy)methyl]morpholine or a pharmaceutically acceptable salt thereof.

**15.** The agent according to claim 13, wherein the compound of the formula (I) or a pharmaceutically acceptable salt thereof is (+)-2-[(inden-7-yloxy)methyl]morpholine or a pharmaceutically acceptable salt thereof.

**16.** The agent according to claim 13, wherein the compound of the formula (I) or a pharmaceutically acceptable salt thereof is (-)-2-[(inden-7-yloxy)methyl]morpholine or a pharmaceutically acceptable salt thereof.

Fig 1

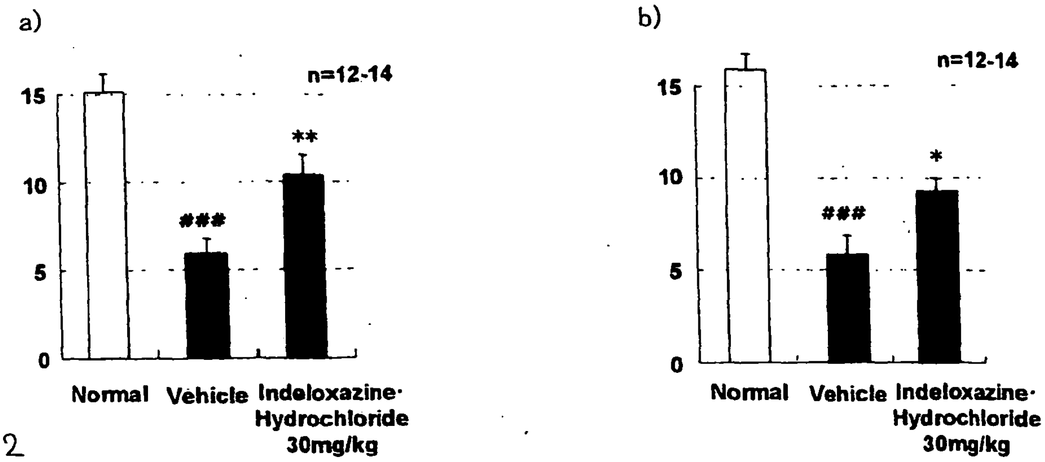


Fig 2

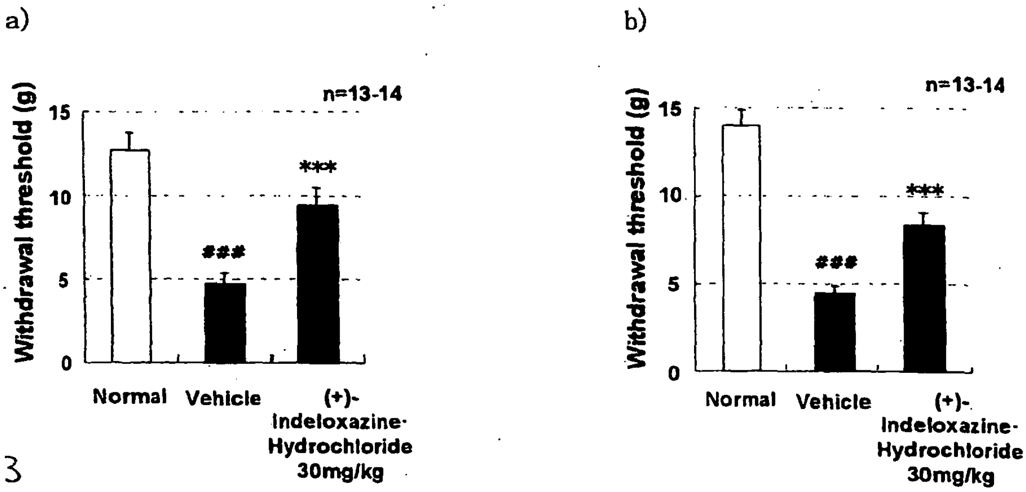


Fig 3

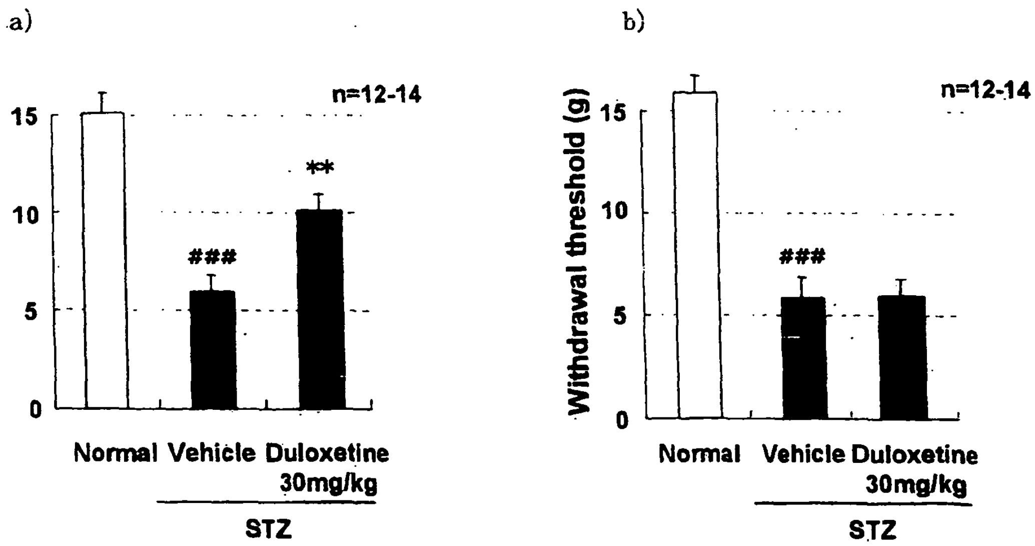

**INTERNATIONAL SEARCH REPORT**

International application No.
PCT/JP2008/054710

A. CLASSIFICATION OF SUBJECT MATTER
*C07D265/30*(2006.01)i, *A61K31/5375*(2006.01)i, *A61P3/10*(2006.01)i, *A61P25/04*(2006.01)i

According to International Patent Classification (IPC) or to both national classification and IPC

B. FIELDS SEARCHED

Minimum documentation searched (classification system followed by classification symbols)
C07D265/30, A61K31/5375, A61P3/10, A61P25/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
BIOSIS(STN), CAplus(STN), EMBASE(STN), MEDLINE(STN), REGISTRY(STN), JSTPlus(JDreamII), JMEDPlus(JDreamII), JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X<br><br>Y | EP 1106184 A2 (Pfizer Products Inc.), 13 June, 2001 (13.06.01), Claims 4, 11, 12 & US 6380200 B1 | 1,2,5,6,13,14<br>3,4,7,8,15,16 |
| X<br><br>Y | WO 2007/017764 A2 (NEURAXON, INC.), 15 February, 2007 (15.02.07), Claims 24, 27, 47 (Family: none) | 1,2,5,6,13,14<br>3,4,7,8,15,16 |
| X<br><br>Y | US 2007/0042969 A1 (RAUSCHKOLB-LOFFLER, C.), 22 February, 2007 (22.02.07), Claims 1, 21 & US 2006/0100157 A1 & EP 1579858 A1 & EP 1737437 A & WO 2005/092313 A | 1,2,5,6,13,14<br>3,4,7,8,15,16 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 April, 2008 (08.04.08) | 22 April, 2008 (22.04.08) |
| Name and mailing address of the ISA/<br>Japanese Patent Office<br>Facsimile No. | Authorized officer<br><br>Telephone No. |

Form PCT/ISA/210 (second sheet) (April 2007)

**INTERNATIONAL SEARCH REPORT**

International application No.
PCT/JP2008/054710

C (Continuation). DOCUMENTS CONSIDERED TO BE RELEVANT

| Category* | Citation of document, with indication, where appropriate, of the relevant passages | Relevant to claim No. |
|---|---|---|
| Y | KOJIMA, T., et al., Synthesis of (±)-2-[(Inden-7-yloxy)methyl]morpholine Hydrochloride (YM-08054, Indeloxazine Hydrochloride) and Its Derivatives with Potential Cerebral-Activating and Antidepressive Properties, Chemical and Pharmaceutical Bulletin, 1985, 33(9), p. 3766-3774 | 1-8,13-16 |
| Y | Kazushige MURAKAWA, Ko Utsu Yaku, Pain Clinicians, 1995, 16(6), pages 814 to 819 | 1-8,13-16 |
| Y | Naoki OTSUKA et al., Ko Utsu Yaku no Chintsu Sayo, Pain Clinicians, 2001, 22(10), pages 1431 to 1436 | 1-8,13-16 |
| Y | JP 2002-524513 A (Eli Lilly and Co.), 06 August, 2002 (06.08.02), Claims 5, 10; Par. No. [0002] & US 6596756 B1 & EP 1113797 A & WO 2000/015223 A1 | 1-8,13-16 |

Form PCT/ISA/210 (continuation of second sheet) (April 2007)

**INTERNATIONAL SEARCH REPORT**

International application No.
PCT/JP2008/054710

**Box No. II Observations where certain claims were found unsearchable (Continuation of item 2 of first sheet)**

This international search report has not been established in respect of certain claims under Article 17(2)(a) for the following reasons:

1. [X] Claims Nos.: 9-12
because they relate to subject matter not required to be searched by this Authority, namely:
The inventions in claims 9 to 12 pertain to methods for treatment of the human body by therapy and thus relate to a subject matter which this International Searching Authority is not required to search (PCT Article 17(2)(a)(i) and PCT Rule 39.1(iv)).

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying additional fees, this Authority did not invite payment of additional fees.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the

[ ] The additional search fees were accompanied by the applicant's protest and, where applicable, payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0015223 A
- US 4109088 A
- US 5521180 A
- US 20070259865 A


### Non-patent literature cited in the description


- **Thomas et al.** *Diabetes,* 1997, vol. 46 (2), S54-S57
- *Diabetes,* 1997, vol. 46 (2), S54-S57
- *Muscle Nerve,* 1988, vol. 11, 21-32
- *Current Opinion in Neurology,* October 2005, vol. 18, 586-590
- *The Informed Prescriber,* December 1996, vol. 11, 122-125
- *Neuropharmacology,* 1998, vol. 37, 1169-1176
- *Chemical and Pharmaceutical Bulletin,* 1985, vol. 33 (9), 3766-3774
- *European Journal of Pharmacology,* 2000, vol. 395 (1), 31-36
- *The Journal of Pharmacology and Experimental Therapeutics,* 2002, vol. 302 (3), 983-991
- *Pain,* 1993, vol. 53, 81-88
- **Cameron et al.** *The Journal of Experimental Physiology,* 1989, vol. 74, 917-926
- **Rae Yuan et al.** *Drug Metabolism and Disposition,* 2002, vol. 30 (12), 1311-1319